# EUROPEAN PATENT APPLICATION

(11) **EP 1 770 095 A1**
(43) Date of publication of application: **04.04.2007**
(21) Application number: 05108856.5
(22) Date of filing: 26.09.2005
(51) Int. Cl.: C07D 493/10

(54) **Efficient catalysts for the asymmetric epoxidation of electron deficient as well as non electron deficient alkenes**

(71) Applicant: Institut Catala D'Investigacio Quimica, 43007 Tarragona (ES); ICREA, Institució Catalana de Recerca I Estudis Avançats, Passeig Lluís Companys, 23 Barcelona CP 08010 (ES)
(72) Inventor: Vidal i Ferran, Anton, Av.Països Catalans, 16 43007 Tarragona (ES); Nieto Alonso, Natàlia, Av. Països Catalans, 16 43007, Tarragona (ES); Molas Porqueras, Pineda Dr., Av. Països Catalans, 16 43007, Tarragona (ES); Benet-Buchholz, Jordi Dr., Av. Països Catalans, 16 43007 Tarragona (ES)
(74) Representative: Barlocci, Anna

(57) **Abstract**

A compound of formula I and its enantiomeric counterpart V; a process for the preparation of diol I, and alternatively diol V, comprising a) the acetylation of diol II, or respectively diol VI, with an acetylating agent, wherein said acetylation is performed in the presence of a Lewis acid and b) an aqueous work-up of the reaction mixture; and a method for producing an enantiomerically enriched epoxide from an alkene, said method comprising admixing said alkene, a substantially enantiomerically pure chiral diol selected from the group comprising diol I and diol V and an oxidizing agent. The method can further comprise the addition of a phase-transfer catalyst.

## Description

### Field of the invention

The present invention is directed to a new efficient catalyst for the asymmetric epoxidation of electron deficient as well as non electron deficient alkenes and a practical preparation process of the same. The present invention is also directed to the enantiomeric counterpart of said catalyst and a practical preparation process of the same.

### Background art

Optically active epoxides are highly versatile intermediates that can be converted into a wide variety of enantiomerically enriched molecules. Among the various methods that have been developed for the preparation of chiral epoxides, the asymmetric epoxidation of alkenes provides a powerful approach to the synthesis of said epoxides.

In recent years, chiral dioxiranes have been shown to be unrivaled agents for the asymmetric epoxidation of specific kinds of alkenes (see for example: Adam, W.; Curci, R.; Edwards, J. O. Acc. Chem. Res.1989, 22, 205-211). Dioxiranes are usually generated from Oxone (potassium peroxomonosulfate) and ketones. The epoxidation can be performed using either isolated dioxiranes or dioxiranes formed in situ from catalytic amounts of a chiral ketone which is regenerated upon epoxidation (see for example: Jacobsen, E. N.; Wu, M. H. Comprehensive Asymmetric Catalysis I-III; 1st ed)

Following the first asymmetric epoxidation using a chiral ketone reported by Curci (Curci, R.; Fiorentino, M.; Serio, M. R. Chem. Commun. 1984, 155-6), numerous chiral ketones have been studied and reported. An important parameter to take into account is the substrate scope. For example, Yang's group has developed chiral ketone **1**, which gave moderate to good enantioselectivity in the epoxidation of trans-olefins and trisubstituted olefins, but not for cis-olefins or terminal olefins (see Dan Yang, Acc. Chem. Res. 2004, 37, 497-505). Outstanding work in the area of asymmetric epoxidation catalysed by chiral ketones has been published by Shi's group, which has led to the development of highly efficient epoxidation catalysts with the general structure 2 (see the European patent EP 1 021 426 B1). Fructose-derived ketone 3, which is commercialy available, is the most-well known asymmetric catalyst from this series, yielding high enantioselectivities in the epoxidation of a wide variety of *trans-* and trisubstituted alkenes (see for example: Shi, Y. Acc. Chem. Res., 2004, 37, 488-496).

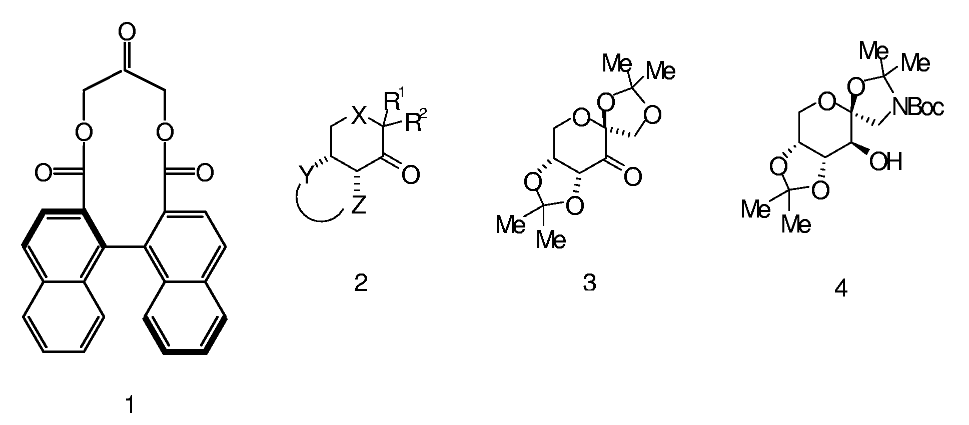

Further modification of the structure led to the discovery of ketone **4**, which efficiently catalyzed enantioselective epoxidations of cis-olefins and terminal olefins.

However, ketones **3** and **4** are not effective towards α,β-unsaturated esters, probably due to the low reactivity of electron-eficient alkenes towards epoxidation and to the decomposition of the catalyst under the reaction conditions. As used herein, the term "electron deficient alkene" refers to an alkene having at least one electron withdrawing group directly bonded to the olefinic moiety. By the term "withdrawing group" is intended "a group with a positive sigma value as defined by Hammett's Equation". Some examples of electron withdrawing groups comprise esters, ketones, sulfones, nitriles and nitro groups. As used herein, the term "non-electron deficient alkene" refers to an alkene having no electron withdrawing group directly bonded to the olefinic moiety.

The only example of catalytic asymmetric epoxidation of electron deficient alkenes by using dioxiranes generated *in situ* has been recently described by Shi (see the international patent application WO 03/066614). Shi has found that chiral ketone **5** is active and highly enantioselective for the catalytic epoxidation of electron deficient alkenes, in particular for the epoxidation of α,β-unsaturated esters.

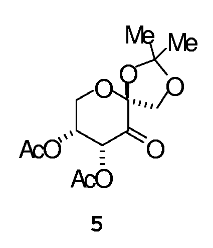

However, the application of chiral ketone **5** has been limited to the catalytic epoxidation of electron deficient alkenes.

Therefore, it is still necessary to find an efficient and robust asymmetric epoxidation catalyst which can be applied to a wide structural variety of alkenes, giving rise to high yields and selectivities and which can be synthesized straightforwardly.

It is an object of the present invention to provide an efficient catalyst which can be applied to the asymmetric epoxidation of electron deficient as well as non electron deficient alkenes. It is also an object of the present invention to provide a practical and scalable preparation process for said catalyst.

### Summary of the invention

It is an object of the present invention to provide an efficient and robust catalyst for the asymmetric epoxidation of a wide variety of alkenes.

The present invention refers to a compound of formula I: 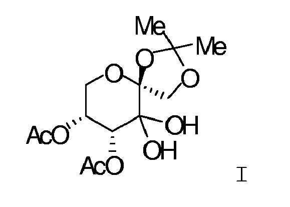 The present invention also refers to a compound of formula V:

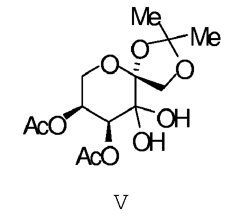

It is a further object of the present invention to provide an efficient preparation process for the asymmetric epoxidation catalyst I, and alternatively, for the asymmetric epoxidation catalyst V.

This object is achieved by the preparation process of the present invention.

The process for the preparation of chiral diol I according to the present invention comprises the following steps:
a) acetylation of diol II with an acetylating agent, characterized in that said acetylation is performed in the presence of a Lewis acid. More specifically, said acetylation is a diacetylation.
b) aqueous work-up of the reaction mixture, yielding chiral diol **I** as a solid material.

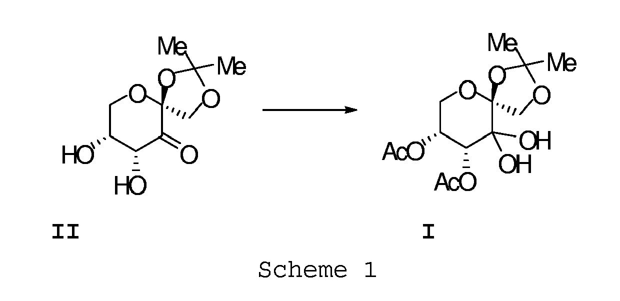

The term "acetylating agent" as defined in the invention refers to a compound capable of providing an acetyl radical through a nucleophilic attack (need of acetylating catalyst). Preferably, said acetylating agent is an acetic acid derivative, more preferably acetic anhydride. According to the preparation process of the present invention, the ratio between the acetylating agent and diol II is from 10:1 to 2:1, preferably 4:1.

Advantageously, the preparation process according to the present invention yields chiral diol I, with high purity, preferably substantially pure, no further purification step being required.

Advantageously, the preparation process of the present invention allows the preparation of chiral diol I with high yield and selectivity. Moreover, the preparation process according to the present invention can be performed in multi-gram scale.

An advantage of the preparation process according to the present invention is that it is performed in mild conditions. Suitable temperatures for the performance of the acetylation reaction according to the present invention range from -10°C to 60°C. In a preferred embodiment of the present invention, said acetylation is performed at room temperature. In a further preferred embodiment of the present invention, said acetylation is performed in a very short period of time under thermal conditions (50°C). In another preferred embodiment of the present invention, said acetylation is performed under microwave irradiation.

A further advantage of the preparation process according to the present invention is that a mild acetylating catalyst is used, more specifically, a Lewis acid. Moreover, only a catalytic amount of Lewis acid is needed. Preferably, said Lewis acid is a group-II metal halide or a group-12 metal halide, most preferably selected between the group comprising zinc halide, magnesium halide and cadmium halide, for example zinc chloride.

In a preferred embodiment, the preparation process of chiral diol I according to the present invention comprises the following steps:
a) selective ketal cleavage on the substantially enantiomerically pure compound III, yielding the substantially enantiomerically pure diol II (see scheme 2);
b) acetylation of diol II with an acetylating agent, wherein said acetylation is performed in the presence of a Lewis acid.
c) aqueous work-up of the reaction mixture.

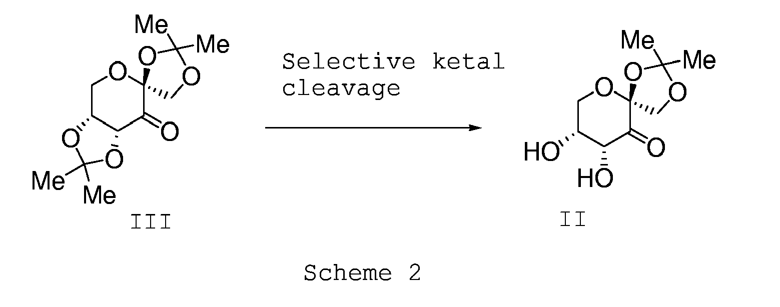

Said selective ketal cleavage can be performed by known procedures, for example the procedure described by Shi and co-workers using substoichiometric amounts of DDQ in hydroorganic media followed by a purification step (Wu, X.-Y.; She, X.; Shi, Y. J. Am. Chem. Soc. 2002, 124, 8792-8793). Alternatively, in a further preferred embodiment of the present invention, said selective ketal cleavage can be performed by reacting compound III with a mixture of acid and water at room temperature, for example by the procedure described by Morris (Morris, P. E., Jr.; Kiely, D. E. J. Org. Chem. 1987, 52, 1149-52).

In a preferred embodiment, the preparation process of chiral diol I according to the present invention comprises the following steps:
a) oxidation of the substantially enantiomerically pure compound IV yielding the substantially enantiomerically pure compound III (see scheme 3);
b) selective ketal cleavage on the substantially enantiomerically pure compound III, yielding the substantially enantiomerically pure diol II (see scheme 2);
c) acetylation of diol II with an acetylating agent, wherein said acetylation is performed in the presence of a Lewis acid.
d) aqueous work-up of the reaction mixture.

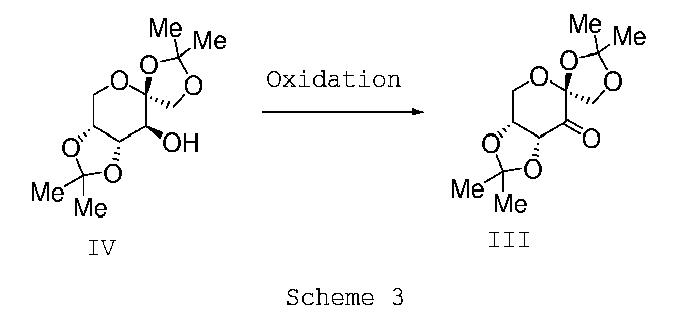

Said oxidation can be performed by known procedures. In a further preferred embodiment of the present invention, said oxidation is promoted by ruthenium, for example by the procedure reported by Mio (Mio, S.; Kumagawa, Y.; Sugai, S. Tetrahedron 1991, 47, 2133-44). Mio has described the quantitative oxidation of compound IV to compound III by using catalytic amounts of a ruthenium precursor and sodium metaperiodate as the stoichiometric oxidant.

In a preferred embodiment, the preparation process of chiral diol I according to the present invention comprises the following steps:
a) reaction of D-fructose with acetone in the presence of acid, yielding the substantially enantiomerically pure compound IV (see scheme 4);
b) oxidation of the substantially enantiomerically pure compound IV yielding the substantially enantiomerically pure compound III (see scheme 3);
c) selective ketal cleavage on the substantially enantiomerically pure compound III, yielding the substantially enantiomerically pure diol II (see scheme 2);
d) acetylation of diol II with an acetylating agent, wherein said acetylation is performed in the presence of a Lewis acid.
e) aqueous work-up of the reaction mixture.

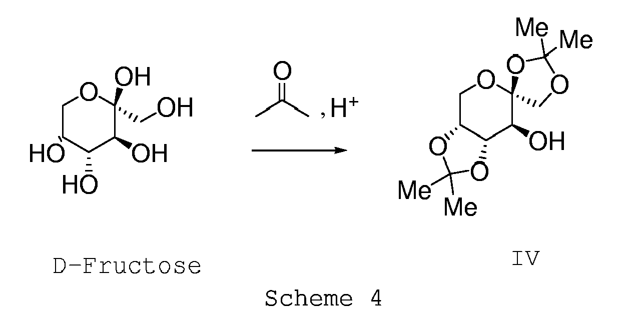

According to this preferred embodiment, chiral diol I is synthesized in four steps from D-fructose. Advantageously, D-fructose is a commercially available inexpensive product. The transformation of D-fructose to compound IV can be carried out through known procedures, for example by the procedure described by Kang and co-workers (Kang, J.; Lim, G. J.; Yoon, S. K.; Kim, M. Y. J. Org. Chem. 1995, 60, 564-577). Kang's procedure was made in acetone as both reagent and solvent at room temperature using sulphuric acid as the catalyst, and the product was purified through recrystallization.

It is a further object of the present invention to provide a preparation process for chiral diol V, the enantiomeric counterpart of chiral diol I.

The process for the preparation of chiral diol V according to the present invention comprises the following steps:
a) acetylation of diol VI with an acetylating agent, characterized in that said acetylation is performed in the presence of a Lewis acid. More specifically, said acetylation is a diacetylation.
b) aqueous work-up of the reaction mixture, yielding chiral diol V as a solid material.

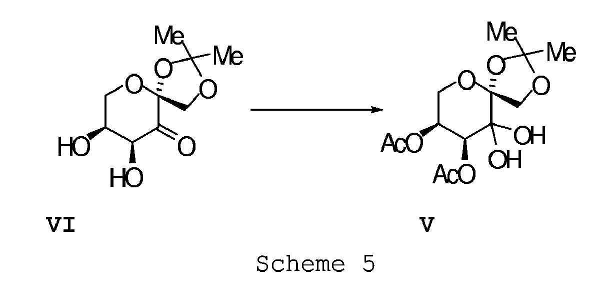

Preferably, the acetylating agent is an acetic acid derivative, more preferably acetic anhydride. According to the preparation process of the present invention, the ratio between the acetylating agent and diol VI is from 10:1 to 2:1, preferably 4:1.

Advantageously, the preparation process according to the present invention yields chiral diol V with high purity, preferably substantially pure, no further purification step being required.

Advantageously, the preparation process of the present invention allows the preparation of chiral diol V with high yield and selectivity.

An advantage of the preparation process according to the present invention is that it is performed in mild conditions. The acetylation reaction according to the present invention can be carried out at a temperature from -10°C to 60°C. In a preferred embodiment of the present invention, said acetylation is performed at room temperature. In another preferred embodiment of the present invention, said acetylation is performed in a very short period of time under thermal conditions (50°C). In a further preferred embodiment of the present invention, said acetylation is performed under microwave irradiation.

A further advantage of the preparation process according to the present invention is that a mild acetylating catalyst is used, more specifically, a Lewis acid. Moreover, only a catalytic amount of Lewis acid is needed. Preferably, said Lewis acid is a group-II metal halide or a group-12 metal halide, most preferably selected between the group comprising zinc halides, magnesium halides and cadmium halides, for example zinc chloride.

In a preferred embodiment, the preparation process of chiral diol V according to the present invention comprises the following steps:
a) selective ketal cleavage on the substantially enantiomerically pure compound VII, yielding the substantially enantiomerically pure diol VI (see scheme 6);
b) acetylation of diol VI with an acetylating agent, wherein said acetylation is performed in the presence of a Lewis acid;
c) aqueous work-up of the reaction mixture.

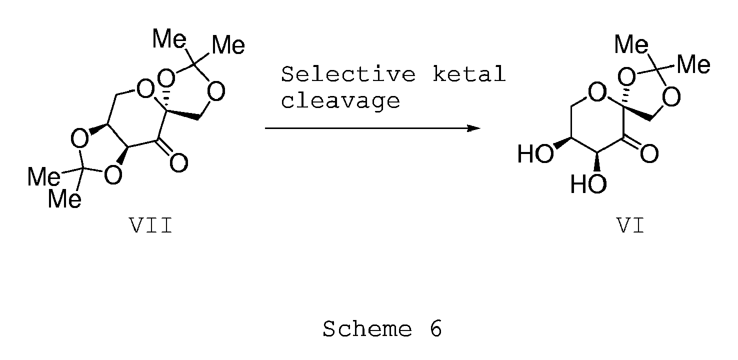

Said selective ketal cleavage can be performed by known procedures, for example the procedure described by Shi and co-workers using substoichiometric amounts of DDQ in hydroorganic media followed by a purification step ( Wu, X.-Y.; She, X.; Shi, Y. J. Am. Chem. Soc. 2002, 124, 8792-8793). Alternatively, in a further preferred embodiment of the present invention, said selective ketal cleavage can be performed by reacting compound VII with a mixture of acid and water at room temperature, for example by the procedure described by Morris (Morris, P. E., Jr.; Kiely, D. E. J. Org. Chem. 1987, 52, 1149-52).

In a preferred embodiment, the preparation process of chiral diol V according to the present invention comprises the following steps:
a) oxidation of the substantially enantiomerically pure compound VIII yielding the substantially enantiomerically pure compound VII (see scheme 7);
b) selective ketal cleavage on the substantially enantiomerically pure compound VII, yielding the substantially enantiomerically pure diol VI (see scheme 6);
c) acetylation of diol VI with an acetylating agent, wherein said acetylation is performed in the presence of a Lewis acid;
c) aqueous work-up of the reaction mixture

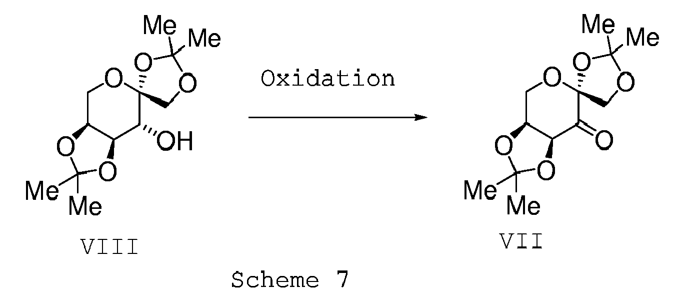

Said oxidation can be performed by known procedures. In a further preferred embodiment of the present invention, said oxidation is promoted by ruthenium, for example by the procedure reported by Mio (Mio, S.; Kumagawa, Y.; Sugai, S. Tetrahedron 1991, 47, 2133-44). Mio has described the quantitative oxidation of compound IV to compound III by using catalytic amounts of a ruthenium precursor and sodium metaperiodate as the stoichiometric oxidant.

Compound VIII can be synthesised through known methods to a person skilled in the art. For example without being a limitation to the scope of the present invention, compound VIII can be synthesised from L-fructose, which in turn can be synthesised, with no limitation to the scope of the present invention, from L-sorbose. Alternatively, without being a limitation to the scope of the present invention, compound VIII can be synthesized from D-arabinose.

In a preferred embodiment, the preparation process of chiral diol V according to the present invention comprises the following steps:
a) reaction of L-fructose with acetone in the presence of acid (see scheme 8), yielding the substantially enantiomerically pure compound VIII;
b) oxidation of the substantially enantiomerically pure compound VIII yielding the substantially enantiomerically pure compound VII (see scheme 7);
c) selective ketal cleavage on the substantially enantiomerically pure compound VII, yielding the substantially enantiomerically pure diol VI (see scheme 6);
d) acetylation of diol VI with an acetylating agent, wherein said acetylation is performed in the presence of a Lewis acid;
e) aqueous work-up of the reaction mixture.

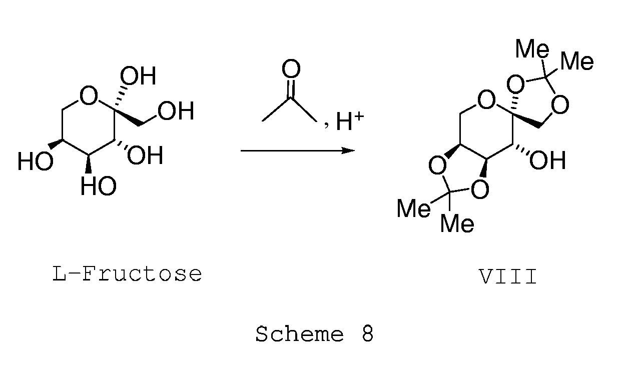

The transformation of L-fructose to compound VIII can be made for example in acetone as both reagent and solvent at room temperature using sulphuric acid as the catalyst.

According to said preferred embodiment, chiral diol V is synthesized in four steps from L-fructose. Advantageously, L-fructose can be synthesised by methods known to a person skilled in the art, for example, with no limitation to the scope of the present invention, from L-sorbose (see Wang, Z.-X.; Tu, Y.; Frohn, M.; Zhang, J.-R.; Shi, Y., J. Am. Chem. Soc., 1997, 119, 11224-11235; see also Chyi-Cheng Chen and Roy L. Whistler, Carbohydr. Res., 1988, 175, 265-271).

In a further preferred embodiment, the preparation process of chiral diol V according to the present invention comprises the following steps:
a) transformation of L-sorbose to L-fructose;
b) reaction of L-fructose with acetone in the presence of acid (see scheme 8), yielding the substantially enantiomerically pure compound VIII;
c) oxidation of the substantially enantiomerically pure compound VIII yielding the substantially enantiomerically pure compound VII (see scheme 7);
d) selective ketal cleavage on the substantially enantiomerically pure compound VII, yielding the substantially enantiomerically pure diol VI (see scheme 6);
e) acetylation of diol VI with an acetylating agent, wherein said acetylation is performed in the presence of a Lewis acid;
f) aqueous work-up of the reaction mixture.

In another preferred embodiment, the preparation process of chiral diol V according to the present invention comprises the following steps:
a) transformation of D-arabinose to compound VIII;
b) oxidation of the substantially enantiomerically pure compound VIII yielding the substantially enantiomerically pure compound VII (see scheme 7);
c) selective ketal cleavage on the substantially enantiomerically pure compound VII, yielding the substantially enantiomerically pure diol VI (see scheme 6);
d) acetylation of diol VI with an acetylating agent, wherein said acetylation is performed in the presence of a Lewis acid;
e) aqueous work-up of the reaction mixture.

Said transformation of D-arabinose to compound VIII can be performed according to methods known to a person skilled in the art (see for example Bessieres, B.; Morin, C., J. Org. Chem., 2003, 68, 4100-4103; see also Chyi-Cheng Chen and Roy L. Whistler, Carbohydr. Res., 1976, 52, 95-101).

In summary, the preparation process of chiral diol I, or alternatively chiral diol V, according to the present invention can be straightforwardly performed and in mild conditions, yielding substantially enantiomerically pure chiral diol I, or alternatively substantially enantiomerically pure chiral diol V, with high yield, chemoselectivity and stereoselectivity. An additional advantage of the preparation process according to the present invention is that a simple aqueous work-up renders analytically pure target chiral diol I, or alternatively, target chiral diol V, no further purification step being required. Thus, chiral diol I, or alternatively, chiral diol V, prepared through the preparation process of the invention can be used without any further purification, for example in the asymmetric epoxidations of interest.

The present invention further relates to a method for producing an enantiomerically enriched epoxide from an alkene, said method comprising admixing said alkene, the substantially enantiomerically pure chiral diol I and an oxidizing agent. The term "enantiomerically enriched" as used herein refers to a sample of a chiral compound whose enantiomeric ratio is greater than 50:50.

Alternatively, the present invention further relates to a method for producing an enantiomerically enriched epoxide from an alkene, said method comprising admixing said alkene, the substantially enantiomerically pure chiral diol V and an oxidizing agent.

Surprisingly, chiral diol I, and alternatively, chiral diol V, can be successfully applied to the catalytic asymmetric epoxidation of alkenes. It has not been described in the background art so far that chiral diols were able to effectively catalyse the asymmetric epoxidation of alkenes. Probably, without being bound to it, diol I effectively catalyses the asymmetric epoxidation of alkenes through the *in situ* generation of dioxiranes.

Moreover, surprisingly, chiral diol I, and alternatively, chiral diol V, catalyse effectively the asymmetric epoxidation of non-electron deficient alkenes as well as electron deficient alkenes. Electron deficient alkenes are less reactive than non-electron deficient alkenes, and thus, most chiral ketones used in the background art as catalysts for the epoxidation of alkenes fail to catalyse the epoxidation of electron-deficient alkenes. On the other hand, more reactive catalysts capable of epoxidating electron-deficient alkenes are not expected by a person skilled in the art to efficiently epoxidate non-electron deficient alkenes, as high reactivity is often associated with low selectivity. Surprisingly, the inventors have found that the epoxidation of non-electron deficient as well as electron deficient alkenes by using chiral diol **I,** or alternatively, chiral diol **V,** as catalyst could be carried out straightforward, with high yield and selectivity.

Chiral diol V shares the features and advantages of chiral diol I with regard to the epoxidation of alkenes and is the enantiomeric counterpart of chiral I, giving rise to epoxides whose optical activity is the opposite of the epoxides obtained in the same reaction conditions using chiral diol I as catalyst. A person skilled in the art will understand that the reactivity features of chiral diol **I** apply to chiral diol **V,** the only difference between said compounds being their opposite stereochemistry and optical activity. The reactions catalysed by chiral diol **I** will give rise to products with the opposite optical activity of those products obtained using chiral diol **V** as catalyst instead of chiral diol **I,** in the same reaction conditions.

In a preferred embodiment of the present invention, said alkene is a non-electron deficient alkene. Suitable non-electron deficient alkenes that can be epoxidated according to the present invention are selected from the group comprising unfunctionalised alkenes, aryl substituted alkenes, hydroxyalkenes, *cis*-alkenes, trans-alkenes and trisubstituted alkenes.

In another preferred embodiment of the present invention, said alkene is an electron deficient alkene.

The method for the asymmetric epoxidation of alkenes according to the present invention can be performed in a variety of different sequences. For example, the oxidation agent can be added to a mixture comprising the chiral diol and the olefin. Alternatively, the actual epoxidation agent (probably, without being bound to it, a dioxirane) can be generated separately prior to contacting with the alkene.

In general, any oxidazing agent capable of providing the actual epoxidation agent (probably, without being bound to it, a dioxirane) from chiral diol I, or alternatively, from chiral diol V, can be used in the present invention. A person skilled in the art will be able to select a suitable oxidizing agent from the group of oxidizing agents described in the background art. However, for economic reasons, a relatively inexpensive oxidizing agent is preferred, such oxidizing agent being selected from the group comprising oxone, peracids, hydrogen peroxide, sodium hypochlorite, potassium peroxomonosulfate, sodium perborate and hypofluoride.

In a preferred embodiment of the method of the present invention, the molar ratio of said chiral diol I, or alternatively, said chiral diol V relative to the amount of said alkene is not higher than 1 equivalent, preferably not higher than 0.3 equivalents, more preferably not higher than 0.1 equivalents.

Typically, any relatively inert solvent can be used in the method of the present invention. In a preferred embodiment, said solvent is a mixture aqueous-organic media. Typically, any relatively inert organic solvent can be used for the present invention, for example acetonitrile-dimethoxymethane.

In a preferred embodiment, the method of the present invention further comprises the addition of a phase-transfer catalyst. Said phase-transfer catalyst can be selected from the group comprising cationic nitrogen containing compounds and cationic phosphorous containing compounds, for example Bu₄NHSO₄.

In a further preferred embodiment, the method of the present invention can further comprise adjusting the pH from pH 6 to pH 8. The pH of the reaction solution can be conveniently achieved by adding sufficient amount of base or buffer to maintain the pH at the desired level. The base or buffer can be added separately, it can be added to the solution containing the compound, or it can be added to the solution containing the oxidizing agent. Any suitable bases and buffers known to a person skilled in the art can be used in the epoxidation method according to the present invention. Without being limited to, a particularly preferred buffer is based on potassium dihydrogen phosphate/potassium hydroxide.

The method of the present invention can be performed at a temperature ranging from -20°C to room temperature, preferably from -10°C to 0°C.

Advantageously, the epoxidation method according to the present invention provides epoxides with at least about 50% yield, more preferably at least about 60% yield and most preferably at least about 70% yield.

Advantageously, the epoxidation method according to the present invention provides epoxides with at least about 70% enantiomeric excess, more preferably about 80% enantiomeric excess and most preferably at least 90% enantiomeric excess.

The findings of the present invention show that chiral diol **I** and chiral diol **V,** respectively, are superior to ketone-based epoxidation catalysts described in the background art, as far as the alkene scope is concerned. More specifically, chiral diol **I** and chiral diol **V** can successfully catalyse the epoxidation of either electron deficient or non electron deficient alkenes.

A further advantage of chiral diol **I** and chiral diol **V** as epoxidation catalysts is related to the catalyst robustness. In particular, chiral diol **I** and chiral diol **V** are not so easily degraded in the reaction media by an undesired Baeyer-Villiger reaction as chiral ketone-based catalysts described in the background art.

A further advantage of chiral diol **I** and chiral diol **V** with regard to chiral ketones used in the epoxidation of alkenes is that chiral diol **I,** and alternatively chiral diol **V** can be easily synthesized in mild conditions through the preparation process of the present invention.

Additional objects, advantages and features of the invention will become apparent to those skilled in the art upon examination of the description or may be learned by practice of the invention. The following drawings and examples are provided by way of illustration and are not intended to limit the scope of the invention. It is intended that the scope of the present invention be defined by the claims appended hereto.

### Brief description of the drawings

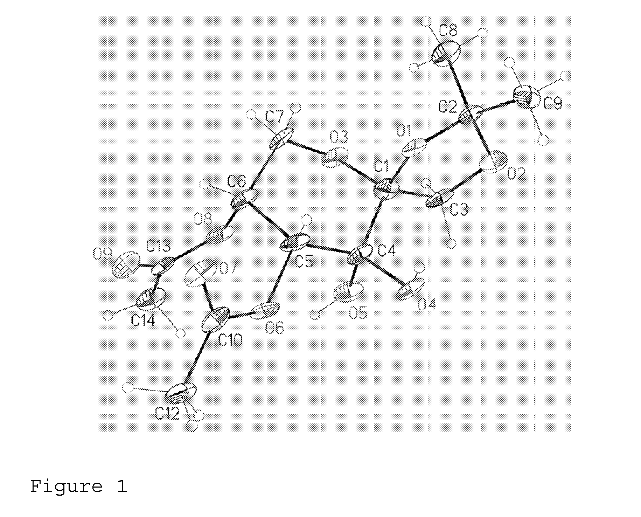

Fig. 1 is the Ortep-plot corresponding to the single crystal X-ray structure analysis of diol **I,** obtained using small crystal needles of diol **I** grown in 1,1,1-trichloroethane.

### Example of preparation of chiral diol I from compound IV

*a) Oxidation:* Compound **IV** (10.0 g, 38.42 mmol), Et₃BzNCl (442 mg, 1.92 mmol), NaIO₄ (12.4 g, 57.24 mmol) and K₂CO₃ (826 mg, 5.92 mmol) were vigorously stirred in a mixture of 33 mL of CHCl₃ and 33 mL of H₂O. RuCl₃ monohydrate (303 mg, 1.34 mmol) was added and the reaction mixture was heated at 70°C. 2-Propanol (11 mL) was added after two hours and the suspension further stirred for 5h. The reaction mixture was filtered through a Celite pad and washed with CH₂Cl₂ (2 x 35 mL). This solution was mixed with the filtrate, the organic layer was separated and the aqueous layer was extracted with CH₂Cl₂ (2 x 20 mL). The combined organic extracts were washed with saturated Na₂SO₃ (130 mL), brine (100 mL) and water (100 mL). Compound **III**, which was obtained after drying and evaporating the solvents as a solid material, was not purified further.
*b) Selective deketalization:* 80 mL of AcOH and 20 mL of water were added at once to the oxidation raw material from the previous step (8.77 g, 38.42 mmol referred to starting material **IV**). The resulting solution was stirred for 12 h at rt. The solvents were removed *in vacuo* at rt and the residue was dissolved in CH₂Cl₂ (50 mL). Compound **II**, which was obtained after drying (an. NaSO₄) and evaporating the CH₂Cl₂ as a solid material, was not purified further.
c) *Acetylation*: ZnCl₂ (132 mg, 0.96 mmol) was added to a suspension of diol **II** arising from step b (8.56 g, 38.42 mmol referred to starting material **IV**) in Ac₂O (19.4 mL, 153.95 mmol) and the mixture stirred under N₂ at room temperature for 3h. 20.0 g of ice were added to the reaction mixture once the acetylation was complete (3h stirring at rt). The precipitate was filtered off the solution, washed with ice-water (2 x 15 mL) and lyophilized to give diol **I** as a white solid (5.14 g, 41% overall yield).

Advantageously, the preparation process according to the present invention leads to compound I in good yield and high purity and it is characterized by mild reaction conditions and easy work-up procedures.
*Characterization of diol I*
[α]^{D}₂₅= -116 (c=0.98, CHCl₃);
mp= 91.1-93.7 °C; IR (ATR) 3467, 1735 cm⁻¹;
¹H-NMR (500 MHz, D₂O) : δ 5.34 (dd, *J*= 4.1, 1.8 Hz, 1H), 5.17 (d, *J*=4.1 Hz, 1H), 4.39 (d, *J*=9.5 Hz, 1H), 4.24 (dd, *J*=13.6, 1.8 Hz, 2H), 4.06 (d, *J*= 9.5 Hz, 1H), 3.85 (dd, *J*= 13.6, 1.8 Hz, 1H), 2.16 (s, 3H), 2.13 (s, 3H), 1.55 (s, 3H), 1.47 (s, 3H);
¹³C-NMR (125 MHz, D₂O) : δ 173.5, 172.7, 113.9, 106.7, 91.2, 70.3, 69.7, 68.7, 61.4, 26.0, 24.9, 20.3, 20.1.
HRMS calcd for C₁₃H₂₀O₉Na 343.10, Found 343.10.
Anal. calcd for C₁₃H₂₀O₉: C 49.00, H 6.51. Found C 48.75, H 6.29.

The structure of diol I has been unambiguously proved by single crystal X-ray structure analysis (see Figure 1).

### Example of asymmetric epoxidation of an alkene by using diol I as catalyst

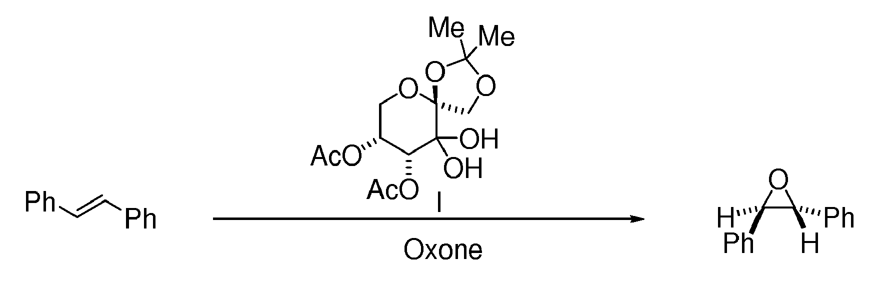

0.4 g (2,22 mmols) of E-stilbene and 72 mg (0,22 mmol) of diol I were dissolved in acetonitrile-dimethoxymethane (44 mL, 1:2 v/v). A pH=6 buffer solution (8 mL), tetrabutylammonium hydrogen sulphate (35 mg, 0.10 mmol) were slowly added with stirring and the mixture was cooled to 0°C. The flask was equipped with two syringe pumps; one of them was filled with a solution of Oxone (2.226 g, 3.62 mmol) in the pH=6 buffer (14 mL) and the other one with a solution of K₂CO₃ (0.744 g, 5.33 mmol) in water (14 mL). The two solutions were added dropwise over a 2h period (syringe pump). The solution was stirred at 0°C for 16 hours. The crude was quenched by addition of water (40 mL) and pentane (10 mL). The reaction mixture was extracted with an organic solvent (hexane, 4 x 40 mL). The combined organic extracts were washed with brine (50 mL), dried over sodium sulphate, filtered and concentrated under reduced pressure. The crude material was purified by flash chromatography on SiO₂, yielding 213 mg (1.09 mmol, 49% yield, 89% ee) of the corresponding (R,R) epoxide. The epoxidation product could be enantiomerically enriched up to 99% ee by recristallization in hexane.

The ee was determined by chiral chromatography and the configuration of the epoxide was established by comparison with reported retention times (chiralpak AD1, (R,R) Rt 5.6 min, (S,S) Rt 8.6 min).

## Claims

1. A compound of formula I:

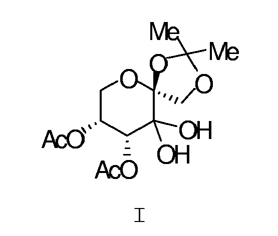

2. A compound of formula V:

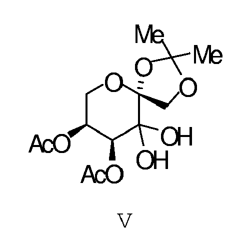

3. A process for the preparation of chiral diol I comprising the following steps:
a) acetylation of diol II with an acetylating agent, wherein said acetylation is performed in the presence of a Lewis acid;
b) aqueous work-up of the reaction mixture.

4. A process for the preparation of chiral diol V, comprising the following steps:
a) acetylation of diol VI with an acetylating agent, wherein said acetylation is performed in the presence of a Lewis acid;
b) aqueous work-up of the reaction mixture.

5. The process according to any of claims 3 or 4, wherein said Lewis acid is a metal halide selected between the group comprising zinc halides, magnesium halides and cadmium halides.

6. A method for producing an enantiomerically enriched epoxide from an alkene, said method comprising admixing said alkene, a substantially enantiomerically pure chiral diol selected from the group comprising diol I and diol V and an oxidizing agent.

7. The method according to claim 6, wherein said alkene is a non electron deficient alkene selected from the group comprising unfunctionalised alkenes, aryl substituted alkenes, hydroxyalkenes, cis-alkenes, trans-alkenes, and trisubstituted alkenes.

8. The method according to claim 6, wherein said alkene is an electron deficient alkene.

9. The method according to any of claims 6 to 8, wherein said oxidizing agent is selected from the group comprising oxone, peracids, hydrogen peroxide, sodium hypochlorite, potassium peroxomonosulfate, sodium perborate and hypofluoride.

10. The method according to any of claims 6 to 9, further comprising the addition of a phase-transfer catalyst.
